(19) Europäisches Patentamt
European Patent Office
Office européen des brevets

(11) **EP 2 627 192 B1**

(12) **FASCICULE DE BREVET EUROPEEN**

(45) Date de publication et mention
de la délivrance du brevet:
**01.07.2015  Bulletin 2015/27**

(51) Int Cl.:
*A23J 3/34* *(2006.01)*          *A23L 1/305* *(2006.01)*
*A61K 38/01* *(2006.01)*          *A23J 1/04* *(2006.01)*
*A61K 35/56* *(2015.01)*          *A61P 25/00* *(2006.01)*

(21) Numéro de dépôt: **11832114.0**

(22) Date de dépôt: **13.10.2011**

(86) Numéro de dépôt international:
**PCT/FR2011/052391**

(87) Numéro de publication internationale:
**WO 2012/049430 (19.04.2012 Gazette 2012/16)**

(54) **HYDROLYSATS DE PROTEINES ANIMALES D'ORIGINE MARINE AUX PROPRIETES NEUROPROTECTRICES**

HYDROLYSATE TIERISCHER PROTEINE MARINEN URSPRUNGS MIT NEUROPROTEKTIVER WIRKUNG

HYDROLYSATES OF ANIMAL PROTEINS OF MARINE ORIGIN WITH NEUROPROTECTIVE PROPERTIES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **14.10.2010  FR 1004043**

(43) Date de publication de la demande:
**21.08.2013  Bulletin 2013/34**

(73) Titulaire: **V. Mane Fils**
**06620 Bar sur Loup (FR)**

(72) Inventeurs:
• **LABATUT, Marie-Luce**
**F-56270 Ploemeur (FR)**
• **LE DENMAT, Solange**
**F-56690 Nostang (FR)**

(74) Mandataire: **de Zeeuw, Johan Diederick**
**Murgitroyd & Company**
**Immeuble Atlantis**
**55, Allée Pierre Ziller**
**Sophia Antipolis**
**06560 Valbonne (FR)**

(56) Documents cités:
**WO-A1-2005/002605      WO-A1-2006/084383**
**WO-A1-2009/101146**

• **VANDANJON L ET AL: "Fractionating white fish fillet hydrolysates by ultrafiltration and nanofiltration", JOURNAL OF FOOD ENGINEERING, BARKING, ESSEX, GB, vol. 95, no. 1, 1 novembre 2009 (2009-11-01), pages 36-44, XP026234382, ISSN: 0260-8774, DOI: DOI: 10.1016/J.JFOODENG.2009.04.007 [extrait le 2009-04-23]**
• **SAMARANAYAKA ET AL: "Autolysis-assisted production of fish protein hydrolysates with antioxidant properties from Pacific hake (Merluccius productus)", FOOD CHEMISTRY, ELSEVIER LTD, NL, vol. 107, no. 2, 19 novembre 2007 (2007-11-19), pages 768-776, XP022351977, ISSN: 0308-8146, DOI: DOI: 10.1016/J.FOODCHEM.2007.08.076**
• **SORENSEN RAYMOND ET AL: "Screening for peptides from fish and cheese inhibitory to prolyl endopeptidase.", NAHRUNG, vol. 48, no. 1, février 2004 (2004-02), pages 53-56, XP002638101, ISSN: 0027-769X**

**Description**

L'invention concerne les traitements visant à réduire les facteurs de risque associés au développement de troubles neurodégénératifs liés au vieillissement, à prévenir l'apparition de ces troubles et/ou à en ralentir le développement. L'invention se rapporte plus particulièrement à des compositions actives destinées à être administrées par voie orale, pour préserver la santé du tissu nerveux d'un individu/patient. Ces compositions, du type complément alimentaire, alicament et médicament, comprennent un hydrolysat de protéines animales d'origine marine possédant des propriétés neuroprotectrices avérées.

L'invention s'étend également à des ingrédients, alimentaires ou pharmaceutiques, destinés à entrer dans la composition de ces compléments alimentaires, alicaments ou médicaments ; ces ingrédients renferment un hydrolysat de protéines animales d'origine marine aux propriétés neuroprotectrices avérées:

Le vieillissement est un processus complexe, lent et progressif, aux effets multiples, qui entraîne un ensemble de modifications anatomiques, biologiques et physiologiques. Il induit une diminution et/ou un dysfonctionnement progressifs des fonctions physiques et/ou intellectuelles d'un individu.

Au niveau du système nerveux, le vieillissement se manifeste par une détérioration progressive du fonctionnement des cellules nerveuses, allant jusqu'à la mort cellulaire. En fonction des régions atteintes, les troubles affecteront la motricité, le langage, la mémoire, le comportement, la concentration, l'humeur... Ces troubles, dits troubles neurodégénératifs (qui, dans certains cas, peuvent correspondre à des démences séniles, à des troubles symptomatiques de la maladie d'Alzheimer, de la maladie de Parkinson...) témoignent de la gravité des atteintes portées au tissu nerveux.

Parmi les facteurs identifiés comme responsables du vieillissement du système nerveux, le stress oxydatif est souvent évoqué. Plus que tout autre organe, le cerveau est particulièrement sensible à l'accumulation de radicaux libres, cause première du stress oxydatif.

Le cerveau possède, en effet, un métabolisme aérobie élevé. Et bien qu'il ne représente que 2 % du poids du corps humain, il utilise plus de 20 % de l'oxygène respiré. Il est ainsi le centre d'une production élevée de radicaux libres. Si dans un état physiologique normal, il parvient à en réguler efficacement le niveau, avec l'âge, cette aptitude s'en trouve diminuée, accentuant le risque d'accidents neurodégénératifs.

L'invention vise à proposer des agents actifs, antioxydants, ayant une efficacité en terme de neuroprotection, et pouvant être prescrits aux fins de prévenir le développement des troubles neurodégénératifs associés au vieillissement et/ou de réduire les facteurs de risque associés au développement de ces troubles. Notamment, l'invention a pour objectif de proposer des agents antioxydants aptes à préserver la santé du tissu nerveux, en particulier à l'égard du stress oxydatif.

Un autre objectif de l'invention est de proposer des agents antioxydants aux effets neuroprotecteurs, qui soient 100 % naturels et efficaces lorsqu'ils sont administrés par voie orale, par exemple, sous la forme d'un médicament *per os,* un complément alimentaire, un alicament. Dans ce même contexte, l'invention vise aussi à proposer des ingrédients intégrant de tels agents antioxydants, qui seront destinés à être utilisés dans la préparation de médicaments, de compléments alimentaires et/ou d'alicaments (c'est-à-dire un aliment prêt à l'emploi ayant des vertus pour la santé).

Les demandes de brevet WO 2006/084383 et WO 2009/101146 décrivent des hydrolysats de protéines provenant du saumon, du maquereau, du tacaud, du cabillaud ou du lieu noir.

L'article par VANDANJON et al., J. Food Engineer., vol. 95, n° 1, 1 novembre 2009, pages 36 - 44, enseigne un hydrolysat de protéines provenant du merlan bleu.

L'article par SAMARANAYAKA et al., Food Chemistry, vol. 107, n° 2, 19 novembre 2007, pages 768 - 776, divulgue l'effet antioxydant d'un hydrolysat de protéines provenant du merlu.

L'article par SORENSEN et al., Nahrung, vol. 48, n° 1, février 2004, pages 53 - 56, observe qu'un hydrolysat de protéines provenant du saumon est capable d'inhiber la prolyle endopeptidase.

L'invention concerne ainsi des hydrolysats de protéines animales d'origine marine possédant des propriétés neuroprotectrices. Ces hydrolysats proviennent d'au moins une source de protéines choisie parmi le maquereau, le saumon, le crabe vert et les poissons blancs (en particulier, le cabillaud, la lingue, le lieu noir, le tacaud, la julienne, le merlan et le merlu).

L'invention résulte ainsi et tout d'abord de la démonstration faite par les inventeurs que des peptides provenant d'animaux marins - en l'occurrence, le maquereau, le saumon, le crabe vert et les poissons blancs - possèdent un effet antioxydant capable d'améliorer la survie de cellules neuronales lorsqu'elles sont soumises à un stress oxydatif.

D'autres hydrolysats de protéines animales d'origine marine, provenant d'autres sources, ont aussi été préparés et testés par les inventeurs, pour leur potentiel neuroprotecteur. Du fait d'une cytotoxicité relativement élevée, aucun d'entre eux n'a cependant été retenu.

L'invention résulte également de la démonstration faite par les inventeurs que des hydrolysats particuliers, conformes à l'invention, présentent non seulement une activité antioxydante neuroprotectrice remarquable, mais aussi une activité inhibitrice de la prolyle endopeptidase (PEP). Cette enzyme, exprimée dans les zones corticales du cerveau humain et dans une moindre mesure au niveau du cervelet, intervient dans le catabolisme de nombre de neurotransmetteurs impliqués dans les processus de la mémoire et de l'apprentissage. Des études menées chez des patients séniors atteints de maladies neurodégénératives ont d'ailleurs permis de mettre en évidence une corrélation entre la perte de la mémoire et une augmentation anormale de l'activité de la PEP. Plus récemment, des travaux ayant conduit à améliorer les fonctions cognitives de rats rendus amnésiques, ont ainsi confirmé l'intérêt grandissant des inhibiteurs de la PEP pour le traitement préventif et/ou curatif des troubles neurodégénratifs liés à l'âge, notamment ceux pour lesquels la perte de mémoire et les troubles cognitifs sont symptomatiques. Dans ce contexte, certains des hydrolysats conformes à l'invention, en particulier les hydrolysats de protéines de maquereau et de lingue, se positionnent en candidats sérieux.

Les hydrolysats de protéines animales d'origine marine aux effets neuroprotecteurs, conformes à l'invention, peuvent être préparés de multiples manières, à partir du maquereau, du saumon, du crabe vert et/ou de poissons blancs (en particulier, le cabillaud, la lingue, le lieu noir, le tacaud, la julienne, le merlan et le merlu). Notamment, ils peuvent être préparés par digestion enzymatique, au moyen d'endopeptidases et/ou d'exopeptidases, par exemple, la papaïne, la subtilisine, l'aminopeptidase, la trypsine, la chymotrypsine... Éventuellement, la digestion enzymatique est précédée d'une étape de broyage mécanique de la source protéique à travailler.

Plus particulièrement, l'invention concerne des hydrolysats de protéines animales d'origine marine préparés à partir du maquereau, du saumon, du crabe vert et/ou de poissons blancs, et dont l'ensemble des peptides présente une distribution en poids moléculaire de profil suivant :

- plus de 20 % de la masse totale des peptides correspondent à des peptides de poids moléculaire inférieur à 300 Da,
- 20 à 35 % de la masse totale des peptides correspondent à des peptides de poids moléculaire compris entre 300 et 500 Da,
- 15 à 35 % de la masse totale des peptides correspondent à des peptides de poids moléculaire compris entre 500 et 1000 Da,
- moins de 15 % de la masse totale des peptides correspondent à des peptides de poids moléculaire supérieur à 1000 Da.

L'invention s'étend à des compositions destinées à être administrées par voie orale, comprenant un hydrolysat de protéines animales d'origine marine tel que décrit précédemment. Lesdites compositions sont avantageusement utilisées/prescrites aux fins de réduire les facteurs de risque associés au développement de troubles neurodégénératifs liés au vieillissement, de prévenir l'apparition de ces troubles et/ou d'en ralentir le développement. Selon l'invention, ces compositions peuvent consister en des médicaments, des compléments alimentaires ou des alicaments.

L'invention s'étend aussi à des ingrédients, alimentaires et/ou pharmaceutiques, comprenant un hydrolysat de protéines animales d'origine marine conforme à l'invention et destinés à être utilisés pour la préparation de ces médicaments, ces compléments alimentaires et/ou ces alicaments.

Quelle que soit la forme dans laquelle elles se présentent (médicament, complément alimentaire, alicament, ingrédient alimentaire), outre ledit hydrolysat de protéines animales d'origine marine tel que décrit précédemment, présent en quantité neuroprotectrice, les compositions selon l'invention peuvent aussi comprendre au moins un additif et/ou au moins un excipient acceptable du point de vue pharmaceutique et/ou alimentaire, ajouté(s) en vue d'obtenir une forme galénique administrable par voie orale. Ces additifs et/ou excipients sont par exemple des liants, des lubrifiants, des édulcorants, des diluants, des excipients, des agents effervescents, des agents d'enrobage...

En particulier, les compositions conformes à l'invention peuvent être réalisées sous des formes galéniques et des formulations diverses, notamment :

- sous la forme d'un mélange de particules solides et divisées, par exemple une poudre effervescente ou non effervescente, et dont la prise nécessite qu'elle soit mélangée à de l'eau,
- sous la forme d'un comprimé effervescent ou non, à avaler avec un peu d'eau, à sucer ou à croquer,
- sous forme d'une solution, par exemple, une suspension, un sirop, une composition liquide pouvant être enrobée dans une capsule dure ou molle.

La présente invention concerne encore l'utilisation des hydrolysats de protéines animales d'origine marine tels que précédemment décrits, pour la fabrication d'un médicament destiné à réduire les facteurs de risque associés au développement de troubles neurodégénératifs liés au vieillissement, à prévenir l'apparition de ces troubles et/ou à en ralentir le développement.

L'invention concerne également des hydrolysats de protéines animales d'origine marine préparés à partir du maquereau, du saumon, du crabe vert et/ou de poissons blancs, ainsi que des compositions - du type complément alimentaire, alicament, médicament ou ingrédient - comprenant une quantité neuroprotectrice desdits hydrolysats de protéines animales d'origine marine, caractérisés en combinaison par tout ou partie des caractéristiques mentionnées ci-dessus ou ci-après.

D'autres buts, caractéristiques et avantages de l'invention apparaîtront à la lecture de la description détaillée qui suit et qui présente, d'une part, des méthodes particulières de préparation de différents hydrolysats conformes à l'invention et, d'autre part, des tests mettant en évidence leur effet neuroprotecteur. Cette description détaillée se veut illustrative et non limitative.

Les résultats expérimentaux obtenus sont notamment présentés sous la forme de représentations graphiques annexées, parmi lesquelles :

- la Figure 1 est une représentation sous forme d'histogrammes, montrant l'innocuité d'hydrolysats selon l'invention, testés sur des cellules Neuro2A ;
- la Figure 2 est une représentation sous forme d'histogrammes, montrant l'effet antioxydant neuroprotecteur d'hydrolysats selon l'invention, testés sur des cellules Neuro2A soumises à un stress oxydatif induit par $H_2O_2$ ;
- la Figure 3 est une représentation sous forme d'histogrammes du pouvoir inhibiteur exercé par des hydrolysats selon l'invention sur la prolyle endopeptidase (PEP).

**Exemple 1 : Préparation d'hydrolysats selon l'invention**

1. Préparation d'un hydrolysat du maquereau

Les maquereaux frais et entiers sont directement soumis à une hydrolyse enzymatique, par la papaïne. Pour ce faire, les maquereaux sont mélangés à de l'eau, dans une proportion [poisson]/[eau] de l'ordre de 2:1. La papaïne est rajoutée au mélange, dans une proportion de l'ordre de 0,12 % par rapport au poids de poisson à traiter. Le pH du mélange est ajusté à 5,8 ± 0,1 au moyen d'acide acétique (80 %), et sa température est maintenue à 52°C. La réaction d'hydrolyse est menée pendant 4 heures sous agitation, puis 4 heures sans agitation.

L'action digestive de la papaïne est stoppée par une élévation de la température du mélange, à environ 85°C. Le mélange est laissé dans cet état, pendant un minimum de 4 heures, sans agitation.

L'hydrolysat de protéines de maquereau obtenu est ensuite filtré de manière à éliminer les insolubles. La filtration est réalisée d'abord sur un tamis rotatif (grille 1 mm) puis au moyen d'un filtre presse. La fraction clarifiée récupérée est pasteurisée à 95°C pendant 1 heure, puis concentrée sous vide.

2. Préparation d'un hydrolysat de pulpe de saumon

Une fois les filets prélevés, la pulpe d'arêtes centrales est récupérée (mécaniquement), affinée puis surgelée, à des fins de conservation jusqu'à l'étape d'hydrolyse enzymatique, réalisée par l'action de la papaïne.

Après broyage, la pulpe est mélangée à de l'eau, dans une proportion [poisson]/[eau] de l'ordre de 1:1. Le mélange est porté à 55°C. La papaïne y est rajoutée, dans une proportion de l'ordre de 0,05 % par rapport au poids de pulpe à traiter.

L'action digestive de la papaïne est stoppée après 3 heures, par une élévation de la température, à environ 85°C. Le mélange est laissé dans cet état, pendant 120 minutes environ.

L'hydrolysat de protéines de saumon obtenu est ensuite filtré de manière à éliminer les insolubles et les résidus d'arêtes. La filtration est réalisée sur un tamis rotatif (grille 1 mm). Une centrifugation permet de parfaire l'élimination des matières insolubles ainsi que l'élimination de la matière grasse (force centrifuge équivalente à un minimum de 1000g).

La phase aqueuse récupérée est pasteurisée à 90°C, pendant 120 à 180 minutes maximum, au delà la température est abaissée à 80°C. L'hydrolysat est alors concentré sous vide.

3. Préparation d'un hydrolysant de crabe vert

Les crabes verts entiers, congelés, sont broyés mécaniquement avant d'être soumis à une hydrolyse enzymatique à la papaïne. Pour ce faire, le broyat de crabe est mélangé à de l'eau, dans une proportion [broyat]/[eau] de l'ordre de 1:1. La papaïne est rajoutée au mélange, dans une proportion de l'ordre de 0,05 % par rapport au poids de crabe à traiter. Le mélange est porté à 58°C et est maintenu à cette température pendant 3 heures.

L'action digestive de la papaïne est alors stoppée en élevant la température du mélange, à environ 85°C. Le mélange est laissé dans cet état, pendant 90 minutes, sous agitation.

Les insolubles du mélange sont éliminés par tamisage sur tamis rotatif (grille 1 mm) puis le jus récupéré est centrifugé afin d'éliminer les matières solubles (force centrifuge équivalente à un minimum de 1000g).

Le surnageant récupéré est pasteurisé à 95°C, pendant 2 à 3 heures, puis concentré sous vide.

4. Préparation d'un hydrolysat de filetages de lingue

Les coproduits de filetages frais ou congelés de poissons sont soumis à une hydrolyse enzymatique, réalisée par une action combinée de la papaïne et de l'alcalase. A cet effet, les coproduits de filetages, broyés ou non, sont mélangés à de l'eau, dans une proportion [poisson]/[eau] de l'ordre de 10:3. Le mélange est porté à 55°C. La papaïne y est rajoutée, dans une proportion de l'ordre de 0,06 % par rapport au poids de poisson à traiter. Pendant la réaction d'hydrolyse, le pH du mélange est ajusté à 5,9 $\pm$ 0,1 à l'aide d'acide phosphorique alimentaire (30%).

Après 90 minutes d'hydrolyse à la papaïne, l'alcalase est ajoutée au mélange, dans une proportion de l'ordre de 0,2 % par rapport au poids de poisson. La réaction d'hydrolyse enzymatique totale durera 8 heures.

L'action digestive de la papaïne et de l'alcalase est stoppée par une élévation de la température, à environ 85°C. Le mélange est laissé dans cet état, pendant 90 minutes.

L'hydrolysat de protéines de lingue obtenu est ensuite filtré de manière à éliminer les insolubles. La filtration est réalisée d'abord sur un tamis rotatif (grille 1 mm) puis à travers un tamis poche (toile 1 mm). Une centrifugation permet de parfaire l'élimination des insolubles (force centrifuge équivalente à un minimum de 1000g).

La fraction soluble récupérée est pasteurisée à 90°C, pendant 90 minutes, la valeur de pH est réajustée à environ 4,8 à l'aide d'acide phosphorique alimentaire (30 %). A la fin de la pasteurisation, le produit est concentré sous vide.

5. Caractérisation physico-chimique des hydrolysats selon l'invention

Les hydrolysats de protéines animales d'origine marine, précédemment préparés, ont été soumis à une analyse physico-chimique. Les résultats obtenus sont portés dans le Tableau 1 ci-après.

Tableau 1

|  | Hydrolysat de maquereau | Hydrolysat de Saumon | Hydrolysat de crabe vert | Hydrolysat de lingue |
|---|---|---|---|---|
| pH à 10 % | 5,9 | 6,5 | 7,1 | 4,9 |
| Extrait sec (%) | 59,7 | 63,0 | 57,2 | 66,2 |
| Protéines / Extrait sec (%) | 91,6 | 70,0 | 78,1 | 80,8 |
| Degré d'hydrolyse (%) | 39,8 | - | 42,7 | 38,6 |
| NaCl / Extrait sec (%) | 3,6 | 19,3 | 17, 7 | |
| Minéraux / Extrait sec (%) | <1 | 25,0 | 22,3 | 9,5 |
| Lipides / Extrait sec (%) | <1 | <5 | | 10,0 |
| Profil peptidique (Da) | | | | |
| PM > 7000 | 0,21 % | 5,54 % | 0,35 % | 0,15 % |
| 7000 > PM > 5000 | 0,07 % | 0,32 % | 0,15 % | 0,07 % |
| 5000 > PM > 3000 | 0,25 % | 0,53 % | 0,68 % | 0,27 % |
| 3000 > PM > 1000 | 5,39 % | 8,85 % | 4,32 % | 7,55 % |
| 1000 > PM > 500 | 25,28 % | 30,19 % | 16,02 % | 31,09 % |
| 500 > PM > 300 | 28,54 % | 29,66 % | 23,81 % | 31,72 % |
| 300 > PM | 40,26 % | 24,94 % | 54,67 % | 29,15 % |
| PM le plus représenté (Da) | 321 | 384 | 263 | 349 |

**Exemple 2 : Évaluation du potentiel neuroprotecteur des hydrolysats selon l'invention**

Chacun des quatre hydrolysats précédemment décrits ont été testés pour leur potentiel neuroprotecteur.

## 1. Mesure de l'effet antioxydant des hydrolysats selon l'invention, sur des cellules Neuro2A

L'effet antioxydant neuroprotecteur des hydrolysats selon l'invention a été évalué au moyen de cellules Neuro2A soumises à un stress oxydatif induit par $H_2O_2$ (300 $\mu$M).

### 1.1. Matériels et méthodes

- Préparation et traitement des cellules Neuro2A

Les cellules Neuro2A sont ensemencées en plaques de culture 96 puits, puis cultivées à 37°C, 5 % $CO_2$ dans le milieu à 10 % de sérum. Après une nuit de culture, les cellules sont rincées au tampon PBS 1X, puis prétraitées pendant 2 heures avec les produits à l'essai ou le témoin positif (Trolox, 10 $\mu$M), dans un milieu de culture dépourvu de sérum. Le stress oxydatif est ensuite induit par l'ajout d'$H_2O_2$ à une concentration finale de 300 $\mu$M. Les cellules sont incubées ainsi pendant 24 heures.

La survie des cellules Neuro2A est alors évaluée via une mesure de leur activité métabolique. Pour ce faire, les cellules, préalablement débarrassées du milieu de traitement, sont incubées durant 2 heures à 37°C et 5 % $CO_2$ avec le MTT bromide. Ce composé est transformé en MTT formazan par les cellules métaboliquement actives. Le MTT formazan produit est ensuite dissout dans le DMSO, puis quantifié par une lecture d'absorbance à 570 nm.

- Préparation des hydrolysats et produits à tester

A partir des hydrolysats bruts à tester et conservés à -18°C, des aliquots sont préparés et conservés à +4°C. A partir de l'aliquot conservé à +4°C, chaque produit à l'essai est dilué dans le milieu de culture des cellules Neuro2A afin d'obtenir une concentration de 100 mg/ml.

Pour les tests, des dilutions intermédiaires sont réalisées.

### 1.2. Évaluation de l'innocuité des hydrolysats selon l'invention, sur les cellules Neuro2A

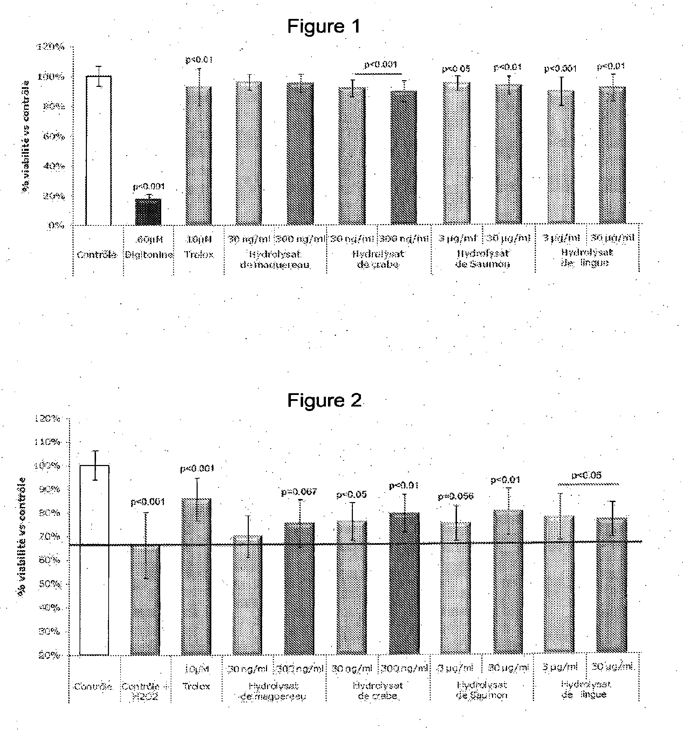

Préalablement à la mesure de leur potentiel antioxydant sur les cellules Neuro2A, les hydrolysats selon l'invention ont été testés pour leur innocuité cellulaire. La Figure 1 présente les résultats obtenus.

La Digitonine à 60 $\mu$M, utilisée comme témoin positif de la toxicité, induit une diminution significative (p<0,001) de la viabilité cellulaire par rapport au contrôle. Ce résultat valide le test de cytotoxicité.

Le Trolox à 10 $\mu$M (témoin positif de protection) ainsi que les hydrolysats selon l'invention ne montrent pas de toxicité ; la viabilité cellulaire est proche de celle du contrôle et est supérieure à 80 %.

### 1.3. Évaluation de l'effet antioxydant des hydrolysats selon l'invention sur les cellules Neuro2A

L'effet neuroprotecteur des hydrolysats à l'essai a été testé sur les cellules Neuro2A soumises à un stress oxydatif induit par $H_2O_2$. La Figure 2 présente schématiquement les résultats obtenus, sous forme d'histogrammes. Des résultats plus détaillés sont consignés dans le Tableau 2 ci-après.

La viabilité du contrôle induit par l'$H_2O_2$ (300 $\mu$M) est significativement diminuée d'environ 35 % par rapport au contrôle non induit, ce qui valide l'induction du stress oxydatif sur l'ensemble des cellules Neuro2A. Le Trolox à 10 $\mu$M permet d'augmenter significativement (p<0,001) la viabilité des cellules Neuro2A ayant subi un stress oxydatif, ce qui valide le test (protection d'environ 20 %).

Au vu des résultats obtenus, il s'avère que la protection des cellules vis-à-vis du stress oxydatif est quelque peu variable d'un hydrolysat testé à l'autre :

- l'effet antioxydant neuroprotecteur de l'hydrolysat de maquereau et de crabe vert apparaît dès la concentration de 0,3 $\mu$g/ml ;
- l'effet antioxydant neuroprotecteur de hydrolysat de lingue est notable à la concentration de 3 $\mu$g/ml ;
- l'effet antioxydant neuroprotecteur de l'hydrolysat de saumon est notable dès la concentration de 3 $\mu$g/ml, et est remarquable à 30 $\mu$M.

Tableau 2

| Produits à l'essai | Dose µg/ml | Cytotoxicité % viabilité (vs contrôle) | p-value (t-test vs contrôle) | Neuroprotect. % viabilité (vs contrôle $H_2O_2$) | p-value (t-test vs contrôle $H_2O_2$) |
|---|---|---|---|---|---|
| Contrôle | | 100 % ± 7 % | - | 100% ±6% | - |
| Contrôlé + $H_2O_2$ | | - | - | 66 % ± 14 % | - |
| Digitonine | 60 µM | 18 % ± 3 % | 0,000 | - | - |
| Trolox | 10 µM | 93% ±12% | 0,003 | 86 % ± 9 % | 0,000 |
| Hydrolysat de maquereau | 0,03 | 96 % ± 5 % | 0,072 | 70 % ± 9 % | 0,530 |
| | 0,3 | 95 % ± 6 % | 0,053 | 76 % ± 10 % | 0,067 |
| Hydrolysat de crabe vert | 0,03 | 92 % ± 6 % | 0,000 | 76 % ± 8 % | 0,040 |
| | 0,3 | 90 % ± 7 % | 0,000 | 80 % ± 8 % | 0,008 |
| Hydrolysat de lingue | 3 | 89% ± 10% | 0,000 | 78 % ± 10 % | 0,025 |
| | 30 | 92 % ± 9 % | 0,003 | 77 % ± 7 % | 0,032 |
| Hydrolysat de saumon | 3 | 95 % ± 5 % | 0,023 | 75% ± 7% | 0,056 |
| | 30 | 94 % ± 6 % | 0,005 | 80 % ± 10% | 0,007 |

**Exemple 3 : Inhibition de la propyle endopeptidase (PEP)**

1. Matériels et méthodes:

Le test enzymatique sur la PEP est réalisé *in tubo,* en plaque de 96 puits. La PEP et les produits à l'essai (hydrolysats, contrôles : Bacitracine et Z-Pro-Prolinal) sont dilués dans le tampon réactionnel aux concentrations souhaitées. Après une incubation de 5 minutes à température ambiante, le substrat (Z-Gly-Pro-pNA) est ajouté à une concentration finale de 160 µM. La réaction enzymatique est alors initiée en plaçant la plaque à 37°C.
L'absorbance de chaque point est mesurée à 410 nm de façon cinétique à 37°C, toutes les 5 minutes pendant 45 minutes. Une condition contrôle sans inhibiteur (substrat + enzyme), ainsi qu'une condition contrôle sans enzyme sont réalisées. Afin de déterminer l'absorbance des produits à l'essai seul, et pour éliminer d'éventuelles interférences, une condition sans enzyme est réalisée pour chaque concentration.

2. Analyse des données

L'inhibition de la PEP est mesurée au bout de 45 minutes. Les valeurs d'absorbance à ce temps sont utilisées pour calculer les pourcentages d'inhibition de la PEP. Les données obtenues pour les produits à l'essai sont normalisées par rapport aux points sans enzyme (« blancs »), puis les pourcentages sont calculés selon la formule :

$$\frac{(A\ produit\ à\ l'essai - A\ contrôle)}{A\ contrôle} \times 100 = pourcentage\ d'inhibition\ de\ la\ PEP$$

$A\ produit\ à\ l'essai$ : absorbance normalisée de chaque point
$A\ contrôle$ : moyenne de l'absorbance des points contrôle (avec enzyme)

Les hydrolysats de maquereau et de lingue, tels que précédemment décrits, ont été analysés pour leur aptitude à pouvoir inhiber la PEP.

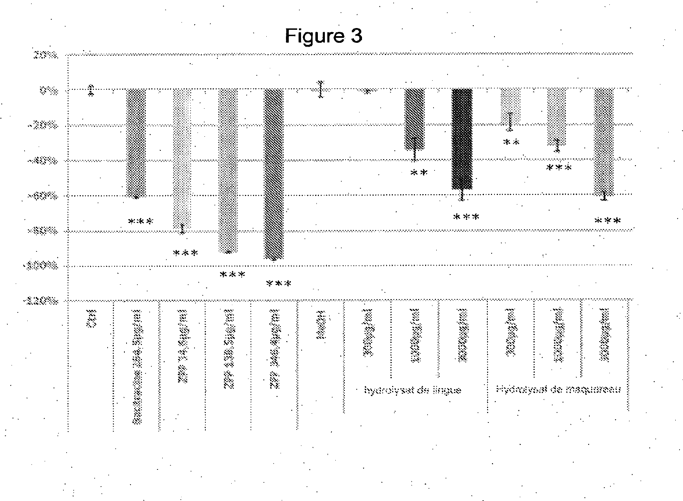

La Figure 3 présente les résultats obtenus, sous forme d'histogrammes. Ces données indiquant que les hydrolysats testés inhibent la PEP de façon significative et dose-dépendante. Aux doses testées, l'inhibition peut atteindre 60 %.

**Revendications**

1.  Hydrolysat de protéines animales d'origine marine, provenant d'au moins une source de protéines choisi parmi le maquereau, le saumon, le crabe vert et les poissons blancs, **caractérisé en ce que** l'ensemble des peptides dudit hydrolysat présente une distribution en poids moléculaire de profil suivant :

    - plus de 20 % de la masse totale des peptides correspondent à des peptides de poids moléculaire inférieur à 300 Da,
    - 20 à 35 % de la masse totale des peptides correspondent à des peptides de poids moléculaire compris entre 300 et 500 Da,
    - 15 à 35 % de la masse totale des peptides correspondent à des peptides de poids moléculaire compris entre 500 et 1000 Da,
    - moins de 15 % de la masse totale des peptides correspondent à des peptides de poids moléculaire supérieur à 1000 Da.

2.  Hydrolysat selon la revendication 1, ledit hydrolysat provenant d'un poisson blanc choisi parmi : le cabillaud, la lingue, le lieu noir, le tacaud, la julienne, le merlan et le merlu.

3.  Complément alimentaire comprenant un hydrolysat de protéines animales d'origine marine selon la revendication 1 ou 2.

4.  Alicament comprenant un hydrolysat de protéines animales d'origine marine selon la revendication 1 ou 2.

5.  Composition pharmaceutique destinée à être administrée par voie orale, comprenant un hydrolysat de protéines animales d'origine marine selon la revendication 1 ou 2.

6.  Ingrédient comprenant un hydrolysat de protéines animales d'origine marine selon la revendication 1 ou 2.

7.  Hydrolysat de protéines animales d'origine marine selon la revendication 1 ou 2 pour son utilisation en tant que médicament.

8.  Utilisation d'un hydrolysat de protéines animales d'origine marine selon la revendication 1 ou 2, pour la fabrication d'un médicament destiné à réduire les facteurs de risque associés au développement de troubles neurodégénératifs liés au vieillissement.

9.  Utilisation d'un hydrolysat de protéines animales d'origine marine selon la revendication 1 ou 2, pour la fabrication d'un médicament destiné à prévenir et/ou ralentir le développement d'un trouble neurodégénératif lié au vieillissement.

10. Utilisation d'un hydrolysat de protéines animales d'origine marine selon la revendication 9, ledit hydrolysat provenant d'au moins une source de protéines choisi parmi le maquereau et la lingue.

**Patentansprüche**

1.  Ein Hydrolysat tierischer Proteine marinen Ursprungs, das aus mindestens einer Proteinquelle stammt, die aus Makrele, Lachs, Strandkrabbe und Weißfischen ausgewählt ist, **dadurch gekennzeichnet, dass** die Gesamtheit der Peptide des Hydrolysats eine Molekülmassenverteilung mit folgendem Profil aufweist:

    - mehr als 20 % der gesamten Peptidmasse entsprechen Peptiden mit einer Molekülmasse von weniger als 300 Da,
    - 20 bis 35 % der gesamten Peptidmasse entsprechen Peptiden mit einer Molekülmasse zwischen 300 und 500 Da,
    - 15 bis 35 % der gesamten Peptidmasse entsprechen Peptiden mit einer Molekülmasse zwischen 500 und 1000 Da,
    - weniger als 15 % der gesamten Peptidmasse entsprechen Peptiden mit einer Molekülmasse von mehr als 1000 Da.

**2.** Hydrolysat gemäß Anspruch 1, wobei das Hydrolysat von einem Weißfisch stammt, der aus Folgenden ausgewählt ist: Kabeljau, Leng, Köhler, Franzosendorsch, Blauleng, Wittling und Seehecht.

**3.** Ein Nahrungsmittelzusatz, der ein Hydrolysat tierischer Proteine marinen Ursprungs gemäß Anspruch 1 oder 2 beinhaltet.

**4.** Ein Nutrazeutikum, das ein Hydrolysat tierischer Proteine marinen Ursprungs gemäß Anspruch 1 oder 2 beinhaltet.

**5.** Eine pharmazeutische Zusammensetzung, die für die orale Verabreichung bestimmt ist und ein Hydrolysat tierischer Proteine marinen Ursprungs gemäß Anspruch 1 oder 2 beinhaltet.

**6.** Ein Inhaltsstoff, der ein Hydrolysat tierischer Proteine marinen Ursprungs gemäß Anspruch 1 oder 2 beinhaltet.

**7.** Hydrolysat tierischer Proteine marinen Ursprungs gemäß Anspruch 1 oder 2 zur Verwendung als Arzneimittel.

**8.** Eine Verwendung eines Hydrolysats tierischer Proteine marinen Ursprungs gemäß Anspruch 1 oder 2 zur Herstellung eines Arzneimittels, das dafür bestimmt ist, die Risikofaktoren, die mit der Entwicklung altersbedingter neurodegenerativer Störungen assoziiert werden, zu reduzieren.

**9.** Verwendung eines Hydrolysats tierischer Proteine marinen Ursprungs gemäß Anspruch 1 oder 2 zur Herstellung eines Arzneimittels, das dafür bestimmt ist, die Entwicklung einer altersbedingten neurodegenerativen Störung zu verhindern und/oder zu verlangsamen.

**10.** Verwendung eines Hydrolysats tierischer Proteine marinen Ursprungs gemäß Anspruch 9, wobei das Hydrolysat aus mindestens einer Proteinquelle stammt, die aus Makrele und Leng ausgewählt ist.

**Claims**

**1.** A hydrolysate of animal proteins of marine origin, originating from at least one source of proteins selected from mackerel, salmon, green crab and white fish, **characterised in that** all of the peptides of said hydrolysate exhibit a molecular weight distribution with the following profile:

- more than 20% of the total mass of the peptides corresponds to peptides of molecular weight lower than 300 Da,
- 20 to 35% of the total mass of the peptides corresponds to peptides of molecular weight of between 300 and 500 Da,
- 15 to 35% of the total mass of the peptides corresponds to peptides of molecular weight of between 500 and 1000 Da,
- less than 15% of the total mass of the peptides corresponds to peptides of molecular weight greater than 1000 Da.

**2.** The hydrolysate according to claim 1, said hydrolysate originating from a white fish selected from: cod, ling, pollock, bib, blue ling, whiting and European hake.

**3.** A food supplement comprising a hydrolysate of animal proteins of marine origin according to claim 1 or 2.

**4.** A nutraceutical comprising a hydrolysate of animal proteins of marine origin according to claim 1 or 2.

**5.** A pharmaceutical composition for oral administration, comprising a hydrolysate of animal proteins of marine origin according to claim 1 or 2.

**6.** An ingredient comprising a hydrolysate of animal proteins of marine origin according to claim 1 or 2.

**7.** The hydrolysate of animal proteins of marine origin according to claim 1 or 2 for use thereof as a medicament.

**8.** Use of a hydrolysate of animal proteins of marine origin according to claim 1 or 2, for the manufacture of a medicament intended for reducing the risk factors associated with the development of ageing-related neurodegenerative disorders.

9. The use of a hydrolysate of animal proteins of marine origin according to claim 1 or 2, for the manufacture of a medicament intended for preventing and/or slowing down the development of an ageing-related neurodegenerative disorder.

10. The use of a hydrolysate of animal proteins of marine origin according to claim 9, said hydrolysate coming from at least one source of proteins chosen from mackerel and ling.

## Figure 1

## Figure 2

## Figure 3

## RÉFÉRENCES CITÉES DANS LA DESCRIPTION

**Documents brevets cités dans la description**

- WO 2006084383 A
- WO 2009101146 A

**Littérature non-brevet citée dans la description**

- **VANDANJON et al.** *J. Food Engineer.,* 01 Novembre 2009, vol. 95 (1), 36-44
- **SAMARANAYAKA et al.** *Food Chemistry,* 19 Novembre 2007, vol. 107 (2), 768-776
- **SORENSEN et al.** *Nahrung,* Février 2004, vol. 48 (1), 53-56